(19) Europäisches Patentamt
European Patent Office
Office européen des brevets

(11) EP 0 884 047 B1

(12) **FASCICULE DE BREVET EUROPEEN**

(45) Date de publication et mention de la délivrance du brevet:
**15.06.2005 Bulletin 2005/24**

(51) Int Cl.[7]: **A61K 7/48**, A61K 7/42, A23L 3/3526

(21) Numéro de dépôt: **98401014.0**

(22) Date de dépôt: **24.04.1998**

# (54) Utilisation de certains polymères polyamines comme agents anti-oxydants

Verwendung von gewissenen Polyaminepolymeren als Antioxidantien

Use of some polyamine polymers as antioxidant agents

(84) Etats contractants désignés:
**DE ES FR GB IT**

(30) Priorité: **22.05.1997 FR 9706258**

(43) Date de publication de la demande:
**16.12.1998 Bulletin 1998/51**

(73) Titulaire: **L'OREAL**
**75008 Paris (FR)**

(72) Inventeurs:
- **Boussouira, Boudiaf**
**75020 Paris (FR)**
- **Colin, Christian**
**75020 Paris (FR)**

(74) Mandataire: **Dodin, Catherine**
**L'OREAL - D.I.P.I.**
**25-29 Quai Aulagnier**
**92600 Asnières (FR)**

(56) Documents cités:
**EP-A- 0 684 044**
**EP-A- 0 816 324**
**WO-A-93/04666**
**WO-A-94/20681**
**WO-A-96/29080**
**WO-A-97/14404**
**FR-A- 2 757 389**
**US-A- 5 449 519**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention se rapporte à l'utilisation d'un polymère polyaminé comme agent antioxydant. Elle concerne en particulier l'utilisation d'un polymère polyaminé pour inhiber la peroxydation photoinduite des lipides. Cette invention concerne notamment l'inhibition de la peroxydation photoinduite de lipides d'origine sébacée (sébum), comme le squalène, ainsi que la peroxydation photoinduite (ou photoperoxydation) des huiles végétales. Elle concerne particulièrement l'inhibition de la photo-peroxydation des lipides induite par les nanopigments. Elle concerne également l'utilisation d'un polymère polyaminé pour inhiber la peroxydation photoinduite des protéines. Cette invention concerne notamment l'inhibition de la peroxydation photoinduite des protéines de la peau, comme le collagène, ainsi que l'inhibition de la peroxydation photoinduite (ou photo-peroxydation) des protéines d'origine animale ou végétale et leurs dérivés. Cette propriété des polymères pglyaminés trouve des applications notamment dans les domaines cosmétique, pharmaceutique, vétérinaire, agroalimentaire.

On sait que les lipides se trouvant à la surface de la peau, du cuir chevelu et des cheveux sont soumis en permanence à des agressions extérieures et notamment l'air, les polluants atmosphériques et les rayonnements visibles et surtout ultraviolets (UV).

Ces lipides sont aussi bien ceux qui font partie des constituants de la peau ou des cheveux, que ceux qui sont sécrétés par la peau y compris le cuir chevelu et/ou ceux qui sont déposés sur la peau ou les cheveux lors de l'application sur ces derniers de produits contenant des lipides.

Les lipides les plus exposés aux agressions extérieures sont ceux contenus dans les sécrétions grasses de la peau comme le sébum, riche en squalène. La présence dans les molécules de squalène de six doubles liaisons rend ces molécules sensibles aux phénomènes d'oxydation. Ainsi, lors d'une exposition prolongée aux UV, le squalène se photoperoxyde pour donner des peroxydes de squalène.

Cette production élevée de peroxydes de squalène provoque notamment une série de dégradations en chaîne en particulier dans et sur la peau, donnant naissance à de nombreux désordres cutanés. Ainsi ces peroxydes de squalène interviennent notamment dans :

- la pathogénèse de l'acné comme décrit par Saint Léger et al. (voir British Journal of Dermatology, 1986 : 114, pp 535-542) qui précisent que les peroxydes de squalène sont comédogènes ;
- le vieillissement prématuré de la peau comme décrit par Keiko OH Sawa et al. (voir The Journal of Toxicology Sciences, 1984 : 19, pp 151-159) qui traitent des conséquences des brûlures cutanées dues au soleil ;
- les phénomènes d'irritation comme rapportés par Takayoshi Tanaka et al. (voir J.Clin.Biochem.Nutr.,1986 : 1, pp 201-207) qui signalent les dommages provoqués notamment par l'utilisation répétée de certains shampooings ;
- la production de produits volatils malodorants (aldéhydes, cétones, acides, etc.) et enfin dans
- l'immunosuppression, comme messagers biochimiques des effets biologiques de l'irradiation UV de la peau, comme décrit par M. PICARDO et al. (voir Photodermatol. Photoimmunol. Photomed., 1991:8, pp 105-110).

On sait que les protéines se trouvant à la surface de la peau, du cuir chevelu et des cheveux sont également soumises à des agressions extérieures et notamment l'air, les polluants atmosphériques et les rayonnements visibles et surtout ultraviolets (UV). A ce sujet, on peut se reporter aux documents suivants : M.-L.HU et A.L.TAPPEL, Photochem. Photobiol. 56 (3), 357-363, 1992 ; M.J.DAVIES et al, Biochem. J., 305, 643-649, 1995.

Ces protéines sont aussi bien celles qui font partie des constituants de la peau ou des cheveux, que celles qui sont sécrétées par la peau y compris le cuir chevelu et/ou celles qui sont déposées sur la peau ou les cheveux lors de l'application sur ces derniers de produits contenant des protéines ou des dérivés de protéines.

Parmi les protéines les plus exposées aux agressions extérieures on peut citer notamment le collagène, les glycoprotéines, les enzymes cutanés.

Afin de limiter la peroxydation des lipides insaturés et des protéines, il est connu d'appliquer sur la peau des compositions photoprotectrices renfermant au moins un agent anti-radicaux libres ou au moins un agent filtrant.

Parmi les agents filtrants, les nanopigments d'oxydes métalliques, particulièrement les dioxydes de titane, sont de plus en plus utilisés dans les produits pour la peau et les cheveux du fait de leurs propriétés de diffusion et de réflexion du rayonnement ultraviolet. Utilisés seuls, ils permettent d'obtenir une bonne protection contre les rayons ultraviolets. Associés à de faibles concentrations de filtres organiques, ils conduisent à l'obtention de produits hautement protecteurs.

Cependant l'irradiation lumineuse des compositions contenant des nanopigments d'oxydes métalliques catalyse l'oxydation des composés organiques sensibles à l'oxydation, notamment :

- des lipides d'origine sébacée (même si on constate un effet globalement protecteur des nanopigments vis-à-vis des lipides d'origine sébacées du fait de leur activité de filtre),
- des lipides utilisés dans les formulations cosmétiques,

- des protéines de la peau,
- des protéines et des dérivés de protéines utilisés dans les formulations cosmétiques.

On observe également une instabilité à la lumière des compositions contenant des nanopigments d'oxydes métalliques, se traduisant notamment pour les oxydes de titane par l'apparition d'une couleur gris-bleuté des produits cosmétiques au contact de la lumière du jour, aussi appelé photobleuissement.
En effet, la demanderesse a mis en évidence, selon la méthode "Head space", que l'oxyde de titane, sous exposition ultraviolette ou après quelques heures à 37 °C, catalyse la production de radicaux peroxydes à partir des constituants lipidiques contenus dans les compositions cosmétiques. Ceci entraîne, lors de l'application sur la peau de ces compositions, des effets nocifs tels que des inflammations. Pour une description de cette méthode, on peut se référer en particulier aux articles de N'GUYEN QL et al. Symposium of AFECG-SFC, Bordeaux mai 1984, pp 358-359, Evaluation de l'oxydation aldéhydique dans les produits cosmétiques ; et de WARNER K et Coll. Journal of Food Science- 1974 V39- pp 761-765, "Pentane formation and rancidity in vegetable oils".
Face à ces désagréments, sont apparus sur le marché des pigments traités en surface, ce traitement ayant pour effet de limiter le phénomène d'activité photoinduite. Ainsi, la demande de brevet WO 90/09777 propose de traiter des nanopigments d'oxyde de titane par des anions phosphate. La demande de brevet JP 03-183620 décrit des nanopigments d'oxyde de zinc traités par une association d'alumine et de silice. Ces traitements de surface visant à réduire l'activité photocatalytique ne sont toutefois pas satisfaisants et la diminution du phénomène reste insuffisante.
Ainsi, les compositions connues à ce jour ne confèrent qu'une protection insuffisante, voire nulle, de la peau vis-à-vis de la peroxydation des lipides et des protéines de la peau et vis-à-vis de la peroxydation des lipides et des protéines contenus dans les compositions cosmétiques destinées à être appliquées sur la peau.
La demanderesse a maintenant découvert que certains polymères polyaminés possédaient des propriétés anti-oxydantes et permettaient de résoudre les problèmes exposés ci-dessus.
L'invention a pour objet des compositions cosmétiques et/ou dermatologiques comprenant dans un support cosmétiquement ou dermatologiquement acceptable, au moins un nanopigment et au moins un polymère polyaminé choisi parmi :

(A)

(i) les polyalkylène polyamines comprenant de 7 à 20000 motifs de répétition et comprenant au moins 5% d'amines tertiaires;
(ii) les dérivés alkylés de polyalkylène polyamines ;
(iii) les produits d'addition d'acides alkylcarboxyliques sur les polyalkylènepolyamines ;
(iv) les produits d'addition de cétones et d'aldéhydes sur les polyalkylènepolyamines ;
(v) les produits d'addition d'isocyanates et d'isothiocyanates sur les polyalkylènepolyamines ;
(vi) les produits d'addition d'oxyde d'alkylène ou de polymères blocs polyoxyde d'alkylène sur les polyalkylènepolyamines ;
(vii) les dérivés quaternisés de polyalkylène polyamines ;
(viii) les produits d'addition d'une silicone sur les polyalkylènepolyamines ;
(ix) un copolymère d'acide dicarboxylique et de polyalkylène polyamines ;

(B) les polyvinylimidazoles ;

(C) les polyvinylpyridines ;

(D) les produits d'addition de monomères 1-vinylimidazole de formule (I) :

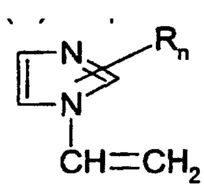

(I)

dans laquelle les radicaux R, identiques ou différents, représentent H, ou un radical alkyle en $C_1$-$C_6$, saturé ou insaturé, linéaire ou cyclique
n est un entier allant de 1 à 3,
avec les polyalkylènepolyamines (A)(i) à (A)(ix) ;

(E) les polymères à base d'acides aminés à chaîne latérale basique ;

(F) les dérivés réticulés des polymères (A)(i) à (A)(ix), (B) (C) (D) et (E).

L'invention a aussi pour objet l'utilisation d'un polymère polyaminé comme agent antioxydant, choisi parmi :

(A) une polyalkylène polyamine, ou un de ses dérivés, choisi parmi :

(i) les polyalkylène polyamines comprenant de 7 à 20000 motifs de répétition et
(ii) les produits d'addition d'acides alkylcarboxyliques sur les polyalkylènepolyamines ;
(iii) les produits d'addition de cétones et d'aldéhydes sur les polyalkylènepolyamines ;
(iv) les produits d'addition d'isocyanates et d'isothiocyanates sur les polyalkylènepolyamines ;
(v) les produits d'addition d'oxyde d'alkylène ou de polymères blocs polyoxyde d'alkylène sur les polyalkylènepolyamines ;
(vi) les dérivés quaternisés de polyalkylène polyamines ;
(vi) les produits d'addition d'une silicone sur les polyalkylènepolyamines
(viii) un copolymère d'acide dicarboxylique et de polyalkylène polyamines ;

(B) les polyvinylimidazoles ;

(C) les polyvinylpyridines ;

(D) les produits d'addition de monomères 1-vinylimidazole de formule (I) :

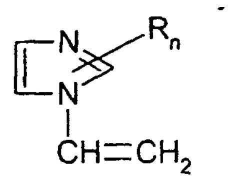

(I)

dans laquelle les radicaux R, identiques ou différents, représentent H, ou un radical alkyle en $C_1$-$C_6$, saturé ou insaturé, linéaire ou cyclique
n est un entier allant de 1 à 3,
avec les polyalkylènepolyamines (A)(i) à (A)(viii) ;

(E) les polymères à base d'acides aminés à chaîne latérale basique ;

(F) les dérivés réticulés des polymères (A)(i) à (A)(viii), (B) (C) (D) et (E).

Les polymères polyaminés utilisables dans la présente invention peuvent se trouver sous la forme de polymère linéaire, de polymère hyperbranché ou de dendrimère.
Les polymères hyperbranchés sont des constructions moléculaires ayant une structure ramifiée, en général autour d'un coeur. Leur structure est en règle générale dépourvue de symétrie : les unités de base ou monomères ayant servi à la construction du polymère hyperbranché peuvent être de natures différentes et leur répartition est irrégulière. Les branches du polymère peuvent être de natures et de longueurs différentes. Le nombre d'unités de base, ou monomères, peut être différent suivant les différentes ramifications. Tout en étant asymétriques, les polymères hyperbranchés peuvent avoir: une structure extrêmement ramifiée, autour d'un coeur ; des couches ou générations successives de ramifications ; une couche de chaînes terminales.
Les polymères hyperbranchés sont généralement issus de la polycondensation d'un ou plusieurs monomères ABx, A et B étant des groupements réactifs susceptibles de réagir ensemble, x étant un entier supérieur ou égal à 2, mais d'autres procédés de préparation peuvent être envisagés. Les polymères hyperbranchés se caractérisent par leur degré de polymérisation DP = 1-b, b étant le pourcentage de fonctionnalités, non terminales, de B qui n'ont pas réagi avec un groupement A. La condensation étant non systématique, au contraire de la synthèse de dendrimères, le degré de polymérisation est inférieur à 100%. De façon habituelle, par les méthodes de synthèses connues, DP est compris entre 15 et 90%. On peut faire réagir un groupement terminal T sur le polymère hyperbranché pour obtenir une fonctionnalité particulière en extrémité de chaînes.
De tels polymères sont décrits en particulier dans B.I.Voit, Acta Polymer., 46, 87-99 (1995) ; EP-682059 ; WO-9614346 ; WO-9614345 ; WO-9612754.

Plusieurs polymères hyperbranchés peuvent être associés entre eux, par une liaison covalente ou un autre type de liaison, par l'intermédiaire de leurs groupes terminaux. De tels polymères dits pontés ou « bridged » entrent dans la définition des polymères hyperbranchés selon la présente invention.

Les dendrimères sont des polymères et oligomères hautement ramifiés, également connus, ayant une structure chimique bien définie, et on dit que ce sont des polymères hyperbranchés « parfaits ». En règle générale, les dendrimères comprennent un coeur, un nombre déterminé de générations de branches, ou fuseaux, et des groupes terminaux. Les générations de fuseaux sont constituées d'unités structurelles , qui sont identiques pour une même génération de fuseaux et qui peuvent être identiques ou différentes pour des générations de fuseaux différentes. Les générations de fuseaux s'étendent radialement en une progression géométrique à partir du coeur. Les groupes terminaux d'un dendrimère de la $N^{ième}$ génération sont les groupes fonctionnels terminaux des fuseaux de la Nième génération ou génération terminale. De tels polymères sont décrits en particulier dans D.A. Tomalia, A.M.Naylor et W.A.Goddard III, *Angewandte Chemie,* Int.Ed.Engl.29, 138-175 (1990); C.J.Hawker et J.M.J.Frechet, *J.Am.Chem.Soc.,* 112, 7638 (1990) ; B.I.Voit, Acta Polymer., 46, 87-99 (1995) ; N.Ardoin et D.Astruc, Bull. Soc. Chim. Fr. 132, 875-909 (1995).

On peut également définir plus particulièrement les dendrimères par la formule (DI) suivante :

$$C[A_1B_1(A_2B_2(...(A_{n-1}\ B_{n-1}(A_nB_n(T)r_n)r_{n-1})r_{n-2}...)r_2)r_1]s \qquad (DI)$$

dans laquelle :

- C représente le coeur, relié par un nombre s de fonctionnalités à s fuseaux $A_1B_1$ par l'intermédiaire des groupements $A_1$ ;
- s est un nombre entier supérieur ou égal à 1 et inférieur ou égal au nombre de fonctionnalités de C ;
- l'indice i (i=1, 2.....n) est un nombre entier qui désigne la génération de chaque fuseau ;
- $r_i$ (i=1, 2.....n-1) représente le nombre de fonctionnalités du groupement $B_i$ appartenant au fuseau ($A_iB_i$), ri étant un entier supérieur ou égal à 2
- pour chaque fuseau ($A_iB_i$) (i=1, 2.....n), le groupement $B_i$ est relié à $r_i$ groupements $A_{i+1}$ d'un fuseau ($A_{i+1}B_{i+1}$) ;
- chaque groupement $A_i$ ($1 \geq 2$) est relié à un seul groupement $B_{i-1}$ du fuseau ($A_{i-1}B_{i-1}$) ;
- le fuseau de $n^{ième}$ génération $A_nB_n$ est chimiquement lié à un nombre $r_n$ de groupes terminaux T, $r_n$ étant un entier supérieur ou égal à zéro.

La définition des dendrimères donnée ci-dessus inclut des molécules à ramifications symétriques ; elle inclut également des molécules à ramification non symétrique, comme par exemple les dendrimères dont les fuseaux sont des groupements lysine, dans lesquels le branchement d'une génération de fuseaux sur la précédente se fait sur les amines $\alpha$ et $\varepsilon$ de la lysine, ce qui conduit à une différence dans la longueur des fuseaux des différentes ramifications.

Les polymères denses en étoiles, ou « dense star polymer », les polymères éclatés en étoile, ou « starburst polymer », les dendrimères en baguette, ou « rod-shaped dendrimer », sont inclus dans la présente définition des dendrimères. Les molécules dénommées arborols et molécules en cascade entrent également dans la définition des dendrimères selon la présente invention.

Plusieurs dendrimères peuvent être associés entre eux, par une liaison covalente ou un autre type de liaison, par l'intermédiaire de leurs groupes terminaux pour donner des entités connues sous le nom de « dendrimères pontés », « agrégats de dendrimères » ou « bridged dendrimer ». De telles entités sont incluses dans la définition des dendrimères selon la présente invention.

Des dendrimères peuvent se présenter sous la forme d'un ensemble de molécules de même génération, ensembles dits monodisperses ; ils peuvent également se présenter sous la forme d'ensembles de générations différentes, dits polydisperses. La définition des dendrimères selon la présente invention inclut des ensembles monodisperses aussi bien que polydisperses de dendrimères.

On peut se reporter aux documents suivants dans lesquels sont décrits des dendrimères comportant des groupements fonctionnels amines : US-4,694,064 ; US-4,631,337 ; WO-A-9502008 ; WO-A-9314147 ; US-4,360,646 ; Proc. Natl. Acad. Sci. USA, 85, 5409-5413 (1988).

Les polymères hyperbranchés et les dendrimères à groupes fonctionnels aminés peuvent également être constitués d'un coeur et de générations d'unités de base, monomères ou fuseaux, de toutes natures, sur lesquels un groupe terminal T porteur d'une fonction amine a été greffé.

On va décrire de façon plus approfondie les polymères polyaminés (A)(i) à (A)(ix), (B), (C), (D), (E) et (F) de l'invention :

(A) (i) les polyalkylène polyamines utilisés selon l'invention sont des polymères contenant de 7 à 20000 motifs de répétition, et comprenant au moins 5% d'amines tertiaires, avantageusement au moins 10% de fonctions amines tertiaires, et encore plus préférentiellement au moins 20%. Ces polymères peuvent être des homopolymères ou des copolymères, linéaires, branchés ou de structures dendrimériques.

Ces polymères comprennent les motifs répétitifs suivants :

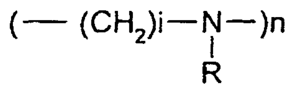

dans lequel :

i représente un entier supérieur ou égal à 2, préférentiellement i=2 ;
n représente un entier
R représente H ou un motif

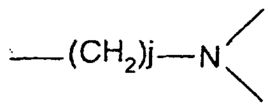

dans lequel j représente un entier supérieur ou égal à 2, préférentiellement j=2 ;

Parmi les produits de la famille des polyalkylène polyamines, appelés aussi polyaziridines, on peut citer en particulier:
La polyéthylèneimine, qui est un polymère hyperbranché bien connu de l'homme du métier: au sujet de la polyéthylèneimine on peut se reporter en particulier aux documents: « KIRK-OTHMER Encyclopedia of Chemical Technology», 3ème édition, vol.20, 1982, p214-216 et "Polyéthylèneimine Prospective Application" H.N. Feigenbaum, Cosmetic & Toiletries, 108, 1993, p 73. La polyéthylèneimine est disponible commercialement auprès de la société BASF sous les noms de marque LUPASOL et POLYIMIN ; la polyéthylèneimine est habituellement comprise dans une gamme de poids moléculaire moyen allant de 500 à 2.000.000 ;

On connaît aussi des polyéthylèneimines et des polypropylèneimines sous la forme de dendrimères, fabriqués par la société DSM. Les demandes de brevet WO 95/02008 et WO 93/14147 décrivent des polyalkylènepolyamines de la famille des dendrimères ainsi qu'un procédé pour leur préparation.
(A)(ii) les dérivés alkylés de polyalkylène polyamine sont des produits bien connus de l'homme du métier. Ils sont obtenus de façon connue par alkylation, en milieu aqueux ou alcoolique, en présence d'un agent alkylant, de préférence en présence de NaOH, de KOH ou de carbonate, à des températures allant de préférence de 40°C à 130°C. L'agent alkylant peut être choisi par exemple parmi des dérivés sulfate d'alkyle ou halogénure d'alkyle en $C_1$-$C_8$, comme par exemple le diméthylsulfate, le diéthylsulfate, le bromure de butyle, le bromure d'hexyle, le bromure de 2-éthylhexyle, le bromure de n-octyle ou les chlorures correspondants. On peut par exemple se reporter à DE-3743744 qui décrit la préparation de tels produits.
(A) (iii) Les produits d'addition d'acides alkylcarboxyliques sur des polyalkylène polyamines sont des produits connus de l'homme du métier et dont la préparation est décrite par exemple dans les demandes de brevet WO 94/14873 WO 94/20681 ; WO 94/12560 dont le contenu est incorporé à la présente demande par référence. L'addition d'acides alkylcarboxyliques sur des polyalkylènepolyamines peut être effectuée en faisant réagir de façon connue un acide, un amide, un ester, un halogénure d'acide sur le polymère polyalkylènepolyamine.

Les produits d'addition d'acides alkylcarboxyliques sur des polyalkylènepolyamines, peuvent être par exemple les produits d'addition d'acides alkylcarboxyliques en $C_2$-$C_{30}$, saturés ou insaturés, linéaires ou ramifiés, sur une polyéthylèneimine. Parmi les acides carboxyliques utilisables, on peut citer par exemple, l'acide acétique, l'acide propionique, l'acide butyrique, l'acide 2-éthylhexanoïque, l'acide benzoïque, l'acide laurique, l'acide myristique, l'acide palmitique, l'acide stéarique, l'acide oléique, l'acide linoléique, l'acide arachidonique, l'acide béhénique, ainsi que les mélanges de corps gras, comme par exemple les mélanges d'esters gras disponibles sous forme de produits naturels, et parmi lesquels on peut citer : l'huile de coco, l'huile de soja, l'huile de lin, l'huile de colza...
(A) (iv) les produits d'addition de cétones et d'aldéhydes sur les polyalkylènepolyamines (A)(i) peuvent être préparés par des procédés connus de l'homme du métier et conduisent à l'obtention de motifs a-hydroxyamine ;
(A) (v) les produits d'addition d'isocyanates et d'isothiocyanates sur les polyalkylènepolyamines (A)(i) peuvent être préparés par des procédés connus de l'homme du métier et conduisent à l'obtention de motifs urée et thio-urée ;

(A) (vi) les produits d'addition d'oxyde d'alkylène ou de polymères blocs polyoxyde d'alkylène sur les polyalkylènepolyamines (A)(i) peuvent être préparés par des procédés connus de l'homme du métier; on peut se reporter par exemple aux documents EP-541018 et US-4,144,123, dans lesquels sont décrites de telles molécules ; des dérivés de polyéthylèneimine éthoxylés sont disponibles commercialement sous le nom de marque : LUPASOL 61(BASF)

(A) (vii) les dérivés quaternisés de polyalkylène polyamines (A)(i) peuvent être préparés par des procédés connus de l'homme du métier ;

(A) (viii) les produits d'addition d'une silicone sur les polyalkylènepolyamines (A)(i) sont par exemple des polyéthylèneimines greffées par des motifs polydiméthylsiloxane dont la préparation est décrite dans le document US-5,556,616 et commercialisées par la société MAC INTYRE sous le nom de marque MACKAMER PAVS

(A) (ix) les copolymères d'acide dicarboxylique et de polyalkylène polyamines (A)(i) peuvent être préparés par polycondensation d'acides dicarboxyliques avec des polyalkylène polyamines.

Parmi les acides dicarboxyliques pouvant être utilisés pour la préparation des polyamidoamines on peut citer les acides dicarboxyliques en $C_2$ à $C_{10}$, comme par exemple l'acide oxalique, l'acide malonique, l'acide itaconique, l'acide succinique, l'acide maléique, l'acide adipique, l'acide glutarique, l'acide sébacique, l'acide téréphtalique, l'acide orthophtalique, ainsi que leurs mélanges.

Les polyalkylène polyamines utilisées pour la préparation des polyamidoamines sont avantageusement choisies parmi celles ayant de 3 à 10 atomes d'azote, comme par exemple la diéthylène tri-amine, la triéthylène tétramine, la dipropylène tri-amine, la tripropylène tétramine, la di-hexaméthylène triamine, l'amino propyléthylène di-amine, la bis-aminopropyléthylène di-amine ainsi que leùrs mélanges. On peut également utiliser des polyéthylène imines telles que décrites ci-dessus pour la préparation de polyamidoamines.

De tels composés sont décrits par exemple dans les documents : US 4,144,423 et WO 94/29422.

(B) le terme polyvinylimidazole comprend les homopolymères et les copolymères de polyvinylimidazole (PVI) obtenus par polymérisation radicalaire des monomères de vinylimidazole de structure suivante :

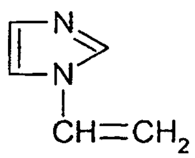

Les copolymères peuvent être par exemple des copolymères de vinylimidazole comportant au moins 5% de motifs vinylimidazole avec des monomères choisis parmi les motifs : vinylpyrrolidinone, acide acrylique et acrylamide. La synthèse de tels composés est bien connue de l'homme du métier; à ce sujet, on peut en particulier se reporter aux documents : J.Am.Chem.Soc., vol.85, 1962, p.951 ; Polymer Letters Ed., vol-11, 1973, p.465-469; Macromolecules, vol.6(2), 1973, p.163-168; Ann.N.Y.Acad.Sci., vol.155, 1969, p.431 ; FR-A-1,477,147 ; JP-69 07395 ; J.Macromol.Scien.Chem., vol.A21(2), 1984, p.253.

(C) le terme polyvinylpyridine comprend les homopolymères et les copolymères de vinyl pyridine obtenus par polymérisation radicalaire des monomères de vinyl pyridine (substitués en position 2 ou 4 du noyau pyridine) de structure suivante :

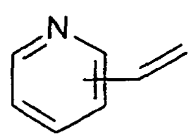

Les copolymères peuvent être par exemple des copolymères de vinylpyridine comportant au moins 5% de motifs vinylpyridine avec des monomères choisis parmi les motifs : vinylpyrrolidinone, acide acrylique et acrylamide.

(D) Les produits d'addition de monomères 1-vinylimidazole répondant à la formule (I) :

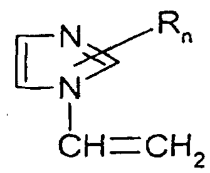 (I)

dans laquelle les radicaux R, identiques ou différents, représentent H, ou un radical alkyle en $C_1$-$C_6$, saturé ou insaturé, linéaire ou cyclique,
n est un entier allant de 1 à 3
avec les polyalkylènepolyamines et leurs dérivés (A)(i) à (A)(ix).

Parmi les dérivés de formule (I) utilisables on peut citer par exemple le 2-méthyl 1-vinyl imidazole, le 2-benzyl 1-vinyl imidazole.

Ces produits sont connus de l'homme du métier: Leur préparation est décrite par exemple dans la demande de brevet WO 94/29422 dont le contenu est incorporé à la présente demande par référence.

(E) Les polymères à base d'acides aminés à chaîne latérale basique sont préférentiellement choisis parmi les protéines et les peptides comprenant au moins 5%, avantageusement au moins 10% d'acides aminés choisis parmi l'histidine, la lysine, l'arginine.
Parmi ces polymères on peut citer par exemple les polylysines, les polyhistidines.

(F) Les dérivés réticulés des polymères (A)(i) à (A)(ix), (B), (C) et (D). Parmi les réticulants utilisables, on peut citer les dérivés halo- hydrin-, glycidyl, aziridino-, isocyanate ; de tels réticulants ainsi que leurs méthodes d'utilisation sont bien connus de l'homme du métier. Parmi les plus connus on peut citer: l'épichlorhydrine, les $\alpha,\omega$-bis-(chlorhydrin) polyalkylène glycoléthers, les $\alpha,\omega$-dichloroalkane comme par exemple le 1,2-dichloroéthane, le 1,2-dichloropropane, le 1,3-dichloropropane, le 1,4-dichlorobutane, et le 1,6-dichlorohexane ; de tels réticulants et leur utilisation pour réticuler des dérivés de polyéthylène imine sont décrits dans WO 94/12560.

On choisit dans la mise en oeuvre de la présente invention des polymères polyaminés comprenant au moins 5% d'amines tertiaires, avantageusement au moins 10% de fonctions amines tertiaires, et encore plus préférentiellement au moins 20%.

Selon l'invention, le polymère polyaminé est choisi avantageusement parmi :

(A)

(i) les polyéthylèneimines hyperbranchées,
(ii) les dérivés alkylés de polyéthylèneimine;
(iii) les produits d'addition d'acides alkylcarboxyliques sur la polyéthylèneimine ;
(iv) les produits d'addition de cétones et d'aldéhydes sur la polyéthylèneimine;
(v) les produits d'addition d'isocyanates et d'isothiocyanates sur la polyéthylèneimine ;
(vi) les produits d'addition d'oxyde d'alkylène ou de polymères blocs polyoxyde d'alkylène sur la polyéthylèneimine;
(vii) les dérivés quaternisés de la polyéthylèneimine ;
(viii) les produits d'addition d'une silicone sur les polyéthylèneimine ;
(ix) les copolymères d'acide dicarboxylique et de polyéthylèneimine ;

(B) les polyvinylimidazoles .

Encore plus préférentiellement, le polymère polyaminé est choisi parmi :

(A) (i) les polyéthylèneimines hyperbranchées ;
(A) (vi) les produits d'addition d'oxyde d'éthylène ou de polymères blocs polyoxyde d'éthylène sur la polyéthylèneimine.

Avantageusement le polymère polyaminé est une polyéthylèneimine.

On choisit des dérivés de polyéthylèneimine comprenant au moins 5% d'amines tertiaires, avantageusement au moins 10% de fonctions amines tertiaires, et encore plus préférentiellement au moins 20%.

De tels polymères permettent d'inhiber les réactions d'oxydation de façon générale, quelle que soit leur origine. En particulier ils ont la propriété d'inhiber la peroxydation photoinduite des lipides photo-oxydables et la peroxydation photoinduite des protéines.

Aussi l'invention a plus particulièrement pour objet l'utilisation d'un polymère polyaminé tel que défini dans la revendication 24 pour inhiber la peroxydation des lipides induite par les rayonnements UV.

Parmi les lipides photo-oxydables concernés par l'invention on peut citer les lipides insaturés du sébum, comme le squalène, ainsi que les corps gras photo-oxydables utilisés habituellement dans la formulation des cosmétiques.

L'utilisation des polymères polyaminés selon l'invention et tels que défini dans la revendication 24 pour inhiber la peroxydation photo-induite des lipides est particulièrement remarquable lorsque ces lipides se trouvent en présence de nanopigments, ces nanopigments ayant la propriété de favoriser les réactions de photo-oxydation.

Ainsi, la présence d'au moins un polymère polyaminé tel que défini dans la revendicatiin 1 dans une composition cosmétique comprenant au moins un nanopigment permet, à l'application sur la peau, d'éviter la photo-peroxydation des dérivés du sébum de la peau.

En outre, la présence d'au moins un polymère polyaminé tel que défini dans la revendication 1 dans une composition cosmétique comprenant au moins un corps gras photo-oxydable et au moins un nanopigment permet, aussi bien au stockage qu'à l'application sur la peau, d'éviter la photo-peroxydation de ces corps gras.

On a également constaté que les polymères polyaminés selon l'invention permettaient d'inhiber la photo-peroxydation des protéines, en particulier la photoperoxydation des protéines induite par les nanopigments.

Aussi l'invention a plus particulièrement pour objet l'utilisation d'un polymère polyaminé tel que défini dans la revendication 24 pour inhiber la peroxydation des protéines induite par les rayonnements UV.

Parmi les protéines photo-oxydables concernées par l'invention on peut citer les protéines de la peau, comme le collagène, ainsi que les protéines photo-oxydables utilisées habituellement dans la formulation des cosmétiques.

L'utilisation des polymères polyaminés tels que défini dans la revendication 24 selon l'invention pour inhiber la peroxydation photo-induite des protéines est particulièrement remarquable lorsque ces protéines se trouvent en présence de nanopigments, ces nanopigments ayant la propriété de favoriser les réactions de photo-oxydation.

Ainsi, la présence d'au moins un polymère polyaminé tel que défini dans la revendication 1 dans une composition cosmétique comprenant au moins un nanopigment permet, à l'application sur la peau, d'éviter la photo-peroxydation des dérivés du collagène de la peau.

En outre, la présence d'au moins un polymère polyaminé tel que défini dans la revendication 1 dans une composition cosmétique comprenant au moins une protéine ou un dérivé de protéine et au moins un nanopigment permet, aussi bien au stockage qu'à l'application sur la peau, d'éviter la photo-peroxydation de ces protéines.

L'invention a également pour objet une composition cosmétique et/ou dermatologique comprenant :

(i) au moins un nanopigment,
(ii) au moins un polymère polyaminé tel que défini dans la revendication 1.

L'invention a en outre pour objet une composition cosmétique et/ou dermatologique comprenant :

(i) au moins un nanopigment,
(ii) au moins un polymère polyaminé tel que défini dans la revendication 1.
(iii) au moins corps gras photo-oxydable.

L'invention a encore pour objet une composition cosmétique et/ou dermatologique comprenant :

(i) au moins un nanopigment,
(ii) au moins un polymère polyaminé tel qeu défini dans la revendication 1
(iii) au moins une protéine ou un dérivé de protéine.

Selon l'invention, on entend par "nanopigment" un pigment de diamètre moyen inférieur à 100 nm et de préférence compris entre 5 et 50 nm.

Plus particulièrement, les nanopigments sont à base d'oxydes métalliques. Les oxydes métalliques sont choisis parmi les oxydes de titane, de zinc, de cérium, de zirconium ou leurs mélanges.
Les nanopigments peuvent être enrobés ou non enrobés.

Les pigments enrobés sont des pigments qui ont subi un ou plusieurs traitements de surface de nature chimique, électronique, mécanochimique et/ou mécanique avec des composés tels que décrits par exemple, dans Cosmetics & Toiletries, Février 1990, vol.105, p.53-64, tels que des aminoacides, de la cire d'abeille, des acides gras, des alcools gras, des tensioactifs anioniques, des lécithines, des sels de sodium, potassium, zinc, fer ou aluminium d'acides gras, des silicones, des protéines (collagène, élastine), des alcanolamines, des oxydes de silicium, des oxydes métalliques ou de l'hexamétaphosphate de sodium, de polyols, d'huiles perfluorées.
Les pigments enrobés sont plus particulièrement des oxydes de titane enrobés :

- de silice tels que le produit "SUNVEIL" de la société IKEDA,
- de silice et d'oxyde de fer tel que le produit "SUNVEIL F" de la société IKEDA,
- de silice et d'alumine tels que les produits "MICROTITANIUM DIOXIDE MT 500 SA" et "MICROTITANIUM DIOXIDE MT 100 SA" de la société TAYCA, "TIOVEIL" de la société TIOXIDE,
- d'alumine tels que les produits "TIPAQUE TTO-55 (B)" et "TIPAQUE TTO-55 (A)" de la société ISHIHARA, et "UVT 14/4" de la société KEMIRA,
- d'alumine et de stéarate d'aluminium tels que le produit "UV TITAN M212" de la société KEMIRA,
- d'alumine et de stéarate d'aluminium tels que le produit "MICROTITANIUM DIOXIDE MT 100 T" de la société TAYCA,
- d'alumine et de laurate d'aluminium tels que le produit "MICROTITANIUM DIOXIDE MT 100 F" de la société TAYCA,
- d'oxyde de zinc et de stéarate de zinc tels que le produit "BR 351" de la société TAYCA,
- de silice, d'alumine et de silicone tels que les produits "MICROTITANIUM DIOXIDE MT SAS" de la société TAYCA,
- de silice, d'alumine et de perfluoropolyméthylisopropyléther tel que le produit "TiO2 VF-25-33" de la société TOSHIKI,
- de silice, d'alumine, de stéarate d'aluminium et de silicone tels que le produit "STT-30-DS" de la société TITAN KOGYO,
- d'alumine et de silicone tels que les produits "TIPAQUE TTO-55 (S)" de la société ISHIHARA, et "UV TITAN M262" de la société KEMIRA,
- de triéthanolamine tels que le produit "STT-65-S" de la société TITAN KOGYO,
- d'acide stéarique tels que le produit "TIPAQUE TTO-55 (C)" de la société ISHIHARA,
- d'hexamétaphosphate de sodium tels que le produit "MICROTITANIUM DIOXIDE MT 150 W" de la société TAYCA.

On peut utiliser également les associations de nanopigments d'oxyde de titane enrobés ou non enrobés rendus dispersibles dans l'eau par un traitement hydrophile ou dispersibles dans les huiles par un traitement hydrophobe telles que celles décrites dans la demande de brevet européen 456 460.

On peut également citer les mélanges d'oxydes métallique, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobés d'alumine, de silice et de silicone tel que le produit "M 261" vendu par la société KEMIRA ou enrobés d'alumine, de silice et de glycérine tel que le produit "M 211" vendu par la société KEMIRA.

Les oxydes de titane non enrobés sont par exemple vendus par la société TAYCA sous les dénominations commerciales "MICROTITANIUM DIOXIDE MT 500 B" ou "MICROTITANIUM DIOXIDE MT 600 B", par la société DEGUSSA sous la dénomination "P 25", par la société WACKHERR sous la dénomination "Oxyde de titane transparent PW", par la société MIYOSHI KASEI sous la dénomination "UFTR", par la société TOMEN sous la dénomination "ITS".

Les oxydes de zinc non enrobés sont par exemple vendus par la société SUMITOMO sous la dénomination "ULTRA FINE ZINC OXIDE POWDER", par la société PRESPERSE sous la dénomination "FINEX 25", par la société IKEDA sous la dénomination "MZO-25" ou par la société SUNSMART sous la dénomination "Z-COTE".

L'oxyde de cérium non enrobé est vendu sous la dénomination "COLLOIDAL CERIUM OXIDE" par la société RHONE POULENC.

Selon l'invention, les nanopigments d'oxyde de titane, enrobés ou non enrobés, sont particulièrement préférés.

La concentration en nanopigments d'oxydes métalliques dans les compositions cosmétiques selon l'invention est comprise entre 0,1 et 20 % en poids par rapport au poids total de la composition, et de préférence entre 0,25 et 15 %.

La concentration en polymères polyaminés dans les compositions cosmétiques selon l'invention est comprise entre 0,05 et 10 % en poids par rapport au poids total de la composition, préférentiellement entre 0,5 et 5%.

L'homme du métier saura ajuster par de simples essais les proportions de polymère polyaminé par rapport au nanopigment, et éventuellement par rapport aux autres composants de la composition, en particulier les corps gras photo-oxydables et les protéines ou les dérivés de protéines. En effet, les proportions optimales des différents constituants peuvent varier, par exemple en fonction du poids moléculaire du polymère, du taux d'amines et/ou du taux d'amines tertiaires dans ce polymère.

Selon l'invention, on entend par corps gras photo-oxydables les corps gras comprenant des chaînes alkyles comportant au moins une insaturation, comme par exemple les acides gras, les acides gras hydroxylés, les stérols et leurs dérivés, parmi lesquels on peut citer les esters de stérols, les esters d'acides gras, les glycolipides, les phospholipides, les céramides et des terpènes.

Les corps gras photo-oxydables concernés par l'invention sont ceux dont l'indice d'iode va de 5 à 200. Pour la mesure de l'indice d'iode, on peut se reporter à "Iodine value : Wij's method, Handbook of Biochem. & mol. Biol. P.512". Cet indice permet de caractériser la susceptibilité des chaînes grasses à s'oxyder.

Les compositions selon l'invention comprennent avantageusement de 0.5% à 60%, en poids par rapport au

poids total de la composition, d'au moins un corps gras photo-oxydable.

On peut donc moduler l'indice d'iode de chacun des composés de la phase grasse par le taux de cet ingrédient dans la phase grasse. On définit le taux d'insaturation C de la phase grasse d'une composition par la formule :

$$C = \sum_{i=1}^{n} [V.I.]_i \cdot [T.H]_i$$

dans laquelle [V.I.]i est l'indice d'iode de l'huile i et [T.H.]i est son pourcentage en poids par rapport au poids total de la phase grasse. Avantageusement le taux d'insaturation C de la phase grasse des compositions selon l'invention est compris entre 2.5 et 4000, et préférentiellement entre 50 et 4000.

L'invention concerne principalement les huiles végétales utilisées en cosmétique, le squalène et le cholestérol présents sur la peau.

Parmi les huiles végétales utilisées en cosmétique on peut citer plus particulièrement les huiles d'abricot, d'amande douce, d'arachide, d'avocat, de bancoulier, de bourrache, de camélia, de camélina, de carthame, de cassis, de céréales, de chouchou, de coco, de colza, de coriandre, de coton, de cumin, de cynara, d'onagre, de perilla, de foie de morue, de germe de mais, de jojoba, de kiwi, de lanoline, de lechti, de lin , de longan, de mangue, de noisette, d'olive, de palme, de passiflore, de pépins de raisin, de pin maritime, de pin parasol, de pistache, de rosier muscat, de sésame, de shoréa (graisse de sol), de soja, de son de riz, de tortue, de tournesol, de baleine, de thé, le beurre de karité les triglycérides de vitamine F.

L'invention concerne également l'inhibition de la photo-peroxydation des protéines : cette propriété s'applique à tout type de protéine, qu'elle soit d'origine animale ou végétale, elle s'applique également aux hydrolysats de protéines (peptides, acides aminés) et aux produits issus de la condensation d'une protéine ou d'un fragment de protéine avec: un acide gras, une silicone, comme par exemple les fibres de collagène sous forme de feuille commercialisés par la société HENKEL sous le nom de marque Polymoist Mask A4-125 ; les hydrolysats de collagène de poissons ou d'aviaires ; les hydrolysats de protéines greffés par des acides gras comme le produit commercialisé par la société SEPPIC sous le nom de marque LIPACIDE ; les hydrolysats de protéines greffés par un fragment polydiméthylsiloxane, comme par exemple le produit commercialisé par la société CRODA sous le nom de marque CRODASONE.

Ces compositions procurent une protection efficace vis-à-vis de toute agression oxydative sans avoir recours à d'autres filtres, notamment chimiques. Ainsi, les risques d'intolérances sont réduits par rapport aux compositions comportant plusieurs filtres. En outre, avec une efficacité équivalente, les compositions selon l'invention sont plus simples à réaliser et moins coûteuses que celles de l'art antérieur.

Les compositions de l'invention peuvent se présenter sous diverses formes galéniques, et en particulier sous forme d'émulsions huile-dans-eau ou eau-dans-huile, de solutions, de gels ou de dispersions vésiculaires. Ces compositions peuvent être une crème de soin, un lait, un shampooing, une lotion, un sérum.

Selon une variante préférée, les compositions de l'invention comprennent, en outre, au moins un agent complexant permettant de complexer les métaux éventuellement présents dans ces compositions et notamment ceux de l'eau utilisée dans la composition et donc de rendre inactifs ces métaux.

L'agent complexant utilisable dans les compositions de l'invention est, par exemple, un dérivé de l'acide phosphonique et est choisi en particulier parmi l'acide 3-éthylènediamine tétra(méthylène phosphonique), l'acide diéthylènetriamine penta(méthylène phosphonique) et leurs sels de sodium. De préférence, l'agent complexant est le sel pentasodique de l'acide diéthylènetriamine penta(méthylènephosphonique).

Comme autres agents complexants neutralisant l'action des métaux, on peut également utiliser l'acide diéthylènetriamine penta-acétique, vendu par exemple par la Société SIGMA.

Les agents complexants, comme l'éthylène diamine tétra-acétique (EDTA) qui est un chélateur du fer, possèdent un effet pro-oxydant et ne peuvent donc pas être utilisés seuls dans les compositions de l'invention à visée antioxydante. Toutefois, il est possible d'utiliser ce type d'agent en association en particulier avec un dérivé d'acide phosphonique. Lorsqu'il est présent, l'agent complexant des métaux a une concentration qui est choisie de 0,005 % à 0,1 % en poids par rapport au poids total de la composition.

Les compositions de l'invention peuvent par ailleurs contenir des adjuvants, utilisés seuls ou en mélange, et en particulier ceux choisis parmi les agents tensioactifs (émulsionnant, ou coémulsionnant), de type non-ionique, anionique, cationique ou amphotère, les agents traitants, les actifs, les épaississants, les agents de mise en suspension, les colorants, les parfums, les charges, les agents neutralisants, les excipients (huiles/eau) et les conservateurs.

La composition de l'invention peut être utilisée comme composition cosmétique ou pour la fabrication d'une composition dermatologique protectrice de l'épiderme humain ou des cheveux contre les rayons ultraviolets, comme composition anti-solaire ou comme produit de maquillage.

Les adjuvants cités peuvent être incorporés aux doses habituelles communément admises en évitant, dans

la mesure du possible, ceux susceptibles de relarguer des métaux catalyseurs d'oxydation. Ils peuvent être lipophiles ou hydrophiles.

Lorsque la composition cosmétique selon l'invention est utilisée pour la protection de l'épiderme humain contre les rayons UV ou comme composition antisolaire, elle peut se présenter sous forme de suspension ou de dispersion dans des solvants ou des corps gras, sous forme de dispersion vésiculaire non ionique ou encore sous forme d'émulsion telle qu'une crème ou un lait, sous forme de pommade, de gel, de bâtonnet solide, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

L'invention se rapporte aussi à une utilisation de ces compositions comme composition cosmétique ou pour la fabrication d'une composition dermatologique destinée au traitement de lutte contre et/ou de prévention des irritations, des inflammations de la peau, de l'acné, de l'immunosuppression, ainsi qu'à l'utilisation de ces compositions pour lutter contre et/ou prévenir les signes du vieillissement. Ces phénomènes cutanés sont notamment induits par le rayonnement solaire.

Elle se rapporte aussi à un procédé de traitement cosmétique de la peau pour lutter contre et/ou prévenir le vieillissement, en particulier le vieillissement photoinduit.

L'invention a encore pour objet un procédé de conservation de compositions contenant au moins un lipide photo-oxydable.

En raison du fait qu'une composition cosmétique et/ou dermatologique comprenant un tel polymère polyaminé bloque instantanément l'amorçage des réactions de peroxydation en présence de rayonnements, une telle composition est particulièrement utile pour lutter contre l'irritation voire l'inflammation de la peau, l'immunosuppression, l'acné photoinduits.

Ainsi l'invention a encore pour objet l'utilisation d'un polymère polyaminé tel que défini dans la revendcation 24 comme agent antioxydant, en particulier dans une composition cosmétique ou pour la préparation d'une composition dermatologique destinée au traitement préventif ou curatif de l'irritation, l'inflammation, l'immunosuppression et/ou l'acné, en particulier l'irritation, l'inflammation, l'immunosuppression et/ou l'acné induits par la photoperoxydation, plus particulièrement la photoperoxydation du squalène et/ou du collagène.

L'invention a en outre pour objet une composition dermatologique selon l'invention, cette composition étant destinée à traiter les signes du vieillissement cutané ou des cheveux, en particulier les signes du vieillissement induits par la photoperoxydation, et plus particulièrement les signes du vieillissement induits par la photoperoxydation du squalène et/ou du collagène.

Dans le cas particulier du traitement de l'acné, on peut également incorporer dans la composition de l'invention, de façon avantageuse, un agent antiacnéïque spécifique, antiséborrhéique et/ou antibactérien, et en particulier, la piroctone olamine, vendue sous la dénomination Octopirox par la société HOECHST.

L'invention a, en outre, pour objet un procédé de traitement cosmétique consistant à lutter contre ou à prévenir les signes du vieillissement par application topique sur la peau et/ou le cuir chevelu et/ou les cheveux d'une composition comprenant au moins un polymère polyaminé selon l'invention. L'invention a, en particulier, pour objet un procédé de traitement cosmétique consistant à lutter contre ou à prévenir les signes du vieillissement induits par la photoperoxydation, et plus particulièrement les signes du vieillissement induits par la photoperoxydation du squalène et/ou du collagène par application topique sur la peau et/ou le cuir chevelu et/ou les cheveux d'une composition comprenant au moins un polymère polyaminé selon l'invention.

De par ses propriétés bénéfiques, la composition de l'invention convient à la protection de tout type de peaux et plus spécialement des peaux grasses et des peaux dites sensibles. Elle peut aussi servir à protéger les lèvres des gerçures.

Parallèlement au traitement topique, la composition de l'invention peut être utilisée pour la conservation de compositions contenant au moins un lipide photo-oxydable.

Ainsi, l'invention a encore pour objet un procédé de conservation de compositions cosmétiques, agro-alimentaires et/ou pharmaceutiques renfermant au moins un lipide ou une protéine ou un dérivé de protéine photo-oxydable qui consiste à incorporer dans lesdites compositions un polymère polyaminé tel que défini ci-dessus.

Selon l'invention, la composition ne présente aucun risque d'intolérance et est utilisable en toute sécurité, notamment dans les domaines cosmétique, dermatologique, voire encore vétérinaire.

La figure 1 illustre l'effet de la polyéthylèneimine sur la photoperoxydation du collagène. En abscisse sont représentés les pourcentages de polyéthylèneimine (en poids par rapport au poids total de la composition) dans la composition soumise au test. En ordonnée sont représentées les quantités de peroxydes de collagène (en équivalents picomoles de $H_2O_2$) dosées dans la composition.

D'autres caractéristiques et avantages de la composition de l'invention ressortiront mieux de la description, des exemples et contre-exemples qui suivent donnés à titre illustratif et non limitatif.
Dans ces exemples et contre-exemples, les pourcentages sont donnés en poids.
Dans les essais décrits ci-dessous, nous avons utilisé la polyéthylèneimine (PEI) de poids moléculaire 700 commercialisée par la société Aldrich.

Essai 1 : Test EX-VIVO d'inhibition de la photo-peroxydation du sébum par la polyéthylèneimine sous UVA

On applique sur le front d'un modèle deux filtres circulaires de 17cm2 pendant 10mn pour extraire un film de sébum. On dépose sur l'un des filtres un film mince de produit placebo (formule A) à raison d'environ 3 mg/cm2. On applique sur le second filtre le produit contenant la polyéthylènimine (produits B à D). Les filtres sont ensuite irradiés sous 5 joules UVA/cm2 à l'aide d'un appareil Biotronic 360.

Les lipides superficiels et les peroxydes sont extraits des filtres, à l'aide de 5 ml d'acétonitrile grade HPLC, en vue du dosage :

- des peroxydes de squalène par détection HPLC-Chimiluminescence après réaction post-colonne à la micropéroxydase-Isoluminol (spécifique des hydropéroxydes) ;
- du squalène résiduel par HPLC diffusion de lumière.

Les résultats sont exprimés en inhibition de la péroxydation du squalène :

$$\%\ inhibition = \frac{\text{SQOOH(formuleA) - SQOOH(formuleX)}}{\text{SQOOH(formuleA)}} \times 100$$

avec :

X=B,C,D ;
SQOOH représente le pourcentage de péroxydes de squalène par rapport au pourcentage de squalène (exprimé en picomoles de péroxydes en équivalent $H_20_2$ par µg de squalène).

Les formules A et X = B, C, D sont des formules de compositions suivantes :

| Nom CTFA | Formule A | Formule B | Formule C | Formule D |
|---|---|---|---|---|
| Cetyl alcohol | 5% | 5% | 5% | 5% |
| Gyceryl stearate | 3% | 3% | 3% | 3% |
| P.E.G. 50 stearate | 3% | 3% | 3% | 3% |
| Mineral oil | 20% | 20% | 20% | 20% |
| Caprilic/Capric triglycerides | 3% | 3% | 3% | 3% |
| Eau | QSP 100 | QSP 100 | QSP 100 | QSP 100 |
| Polyéthylènimine | 0% | 0,2% | 0,5% | 1% |

On obtient les résultats d'inhibition suivants :

| Formule | Inhibition de la peroxydation |
|---|---|
| A | 0% |
| B | 40% |
| C | 76% |

(suite)

| Formule | Inhibition de la peroxydation |
|---|---|
| D | 84% |

Essai 2 : Inhibition par la polyéthylèneimine de la photo-oxydation de la vitamine F sous UVA induite par des nanopigments de TiO2 photo-réactifs

Principe du test : des compositions (A',B',C') comprenant de la vitamine F, de l'huile de jojoba et du $TiO_2$ et éventuellement de la polyéthylèneimine sont étalées sur un filtre. On initie une dégradation photo-oxydative de la vitamine F et de l'huile de jojoba sous l'action conjointe des nanopigments de $TiO_2$ et d'une irradiation par UVA de 100 joules par cm2 à l'aide d'un appareil Suntest Heareus CPS. Cette dégradation conduit à la formation de pentane qui est utilisé comme marqueur de cette oxydation. La formation de pentane est mesurée par chromatographie à l'aide d'un appareil Head-Space-40 (commercialisé par la société Perkin-Elmer). On suit donc l'action de la PEI sur la formation du pentane.

Les compositions des formules sont les suivantes :

| Nom CTFA | Formule A' | Formule B' | Formule C' |
|---|---|---|---|
| Cetearyl alcohol | 4% | 4% | 4% |
| Ceteareth-33 | 1% | 1% | 1% |
| Glyceryl stearate | 1% | 1% | 1% |
| Cetyl alcohol | 1% | 1% | 1% |
| Jojoba oil | 6% | 6% | 6% |
| Vitamine F | 6% | 6% | 6% |
| $TiO_2$ P25 de Degussa | 0% | 5% | 5% |
| PEI | 0% | 0% | 2% |
| Eau | QSP à 100% | | |

On obtient les résultats suivants :

| Formules | pentans (en ppm) |
|---|---|
| Formule A' | 9 |
| Formule B' | 53 |
| Formile C' | 10 |

La formule C' inhibe la formation du pentane avec une efficacité d'environ 80% par rapport à la formule B'. Elle est presque équivalente au témoin (formule A') qui ne contient pas de nanopigments, ce qui correspond à une inhibition de 97% du phénomène oxydatif imputable à l'action des nanopigments.

Essai 3 : Inhibition de la photoperoxydation du collagène par la polyéthylèeimine sous UVA

Principe du test : Ce test permet d'évaluer la formation des résidus peroxydes au sein des fibres de collagène sous irradiation UVA (365 nm). On irradie le collagène (feuilles de collagène commercialisées par la société Henkel sous le nom de marque Polymoist mask) à l'aide d'un radiomètre programmable (Biotronic UV commercialisé par la société Vilbert Lourmat muni de 3 tubes à vapeur de mercure basse pression 40 W /365 nm).
On mesure par chimiluminescence les peroxydes formés grâce à une caméra compteuse de photons (Caméra CCD commercialisée par la société Hamamatsu): les peroxydes sont décomposés par une enzyme, la microperoxydase (Microperoxydase MP 11 10 H2O commercialisée par la société Sigma). Lors de cette réaction, des espèces activées oxygénées sont produites et vont réagir avec le 6-amino-2,3-dihydro-1,4-phtalazinedione (Isoluminol, commercialisé par la société Sigma) également présent dans le milieu réactionnel. C'est à l'issue de cette réaction que l'isoluminol va émettre des photons en se désactivant. Les photons émis, sont comptés par la caméra, leur nombre est proportionnel

à la quantité de peroxydes initialement présents.

Formules testées :

| Nom CTFA | Formule 1 (placébo) | Formule 2 | Formule 3 | Formule 4 |
|---|---|---|---|---|
| Cetyl alcohol | 5% | 5% | 5% | 5% |
| Gyceryl stearate | 3% | 3% | 3% | 3% |
| P.E.G. 50 stearate | 3% | 3% | 3% | 3% |
| Mineral oil | 20% | 20% | 20% | 20% |
| Caprilic/Capric triglycerides | 3% | 3% | 3% | 3% |
| Eau | QSP 100 | QSP 100 | QSP 100 | QSP 100 |
| Polyéthylènimine | 0% | 0.5% | 1% | 2% |

Mode Opératoire :

Préparation du mélange réactionnel de chimiluminescence :
On pèse 34.8 mg d'Isoluminol et on le solubilise sous agitation magnétique dans environ 50 ml de tampon Borate 100 mM. On pèse 2 mg de Microperoxydase et on le solubilise dans environ 5 ml de tampon Borate 100 mM. Les deux réactifs sont alors mélangés dans une fiole jaugée de 200 ml dont le volume est ajusté par du tampon Borate. Les 200 ml sont placés dans un flacon de 500 ml. 200 ml de tampon Borate y sont alors à nouveau ajoutés. Le mélange réactionnel, dont le volume final est alors de 400 ml, est homogénéisé par agitation magnétique puis placé à l'obscurité au moins 24 heures avant emploi.

Des pastilles de diamètre 6 mm sont découpées à l'emporte pièce dans une feuille de collagène. Ces dernières sont alors placées dans des boites de pétri en verre, soit en solution aqueuse, soit sur un gel d'agarose à 1%. On applique les formules 1 à 4 sur chaque échantillon de collagène. Pour chaque formule testée, 6 pastilles sont utilisées par boite. Chaque formule à tester est appliquée à l'aide d'un doigtier de façon à saturer la pastille de collagène. Les boites de pétri sont alors exposées à des UVA (365 nm) d'intensité : 10 Joules/cm2. A l'issue de l'irradiation, les pastilles de collagène sont placées dans une plaque 96 puits, dans un caisson étanche à la lumière, sous une caméra compteuse de photons.
150 microlitres du mélange réactionnel de chimiluminescence sont alors répartis dans chacun des puits impliqués dans la mesure. Dans le même temps, le comptage de la caméra photon est déclenché. Le comptage des photons est pratiqué sur un temps de 4 minutes.
Les valeurs de comptage obtenues sont alors converties en équivalents picomoles d'Hydroproxyde ($H_2O_2$) par régression linéaire effectuée à partir d'une gamme étalon.

Les résultats sont illustrés par la figure 1. On constate que la polyéthylèneimine inhibe la formation de peroxydes de collagène.

## Revendications

1. Composition cosmétique et/ou dermatologique comprenant dans un support cosmétiquement ou dermatologiquement acceptable, au moins un nanopigment et au moins un polymère polyaminé choisi parmi :

    (A)

    (i) les polyalkylène polyamines comprenant de 7 à 20000 motifs de répétition et comprenant au moins 5% d'amines tertiaires;
    (ii) les dérivés alkylés de polyalkylène polyamines ;
    (iii) les produits d'addition d'acides alkylcarboxyliques sur les polyalkylènepolyamines ;
    (iv) les produits d'addition de cétones et d'aldéhydes sur les polyalkylènepolyamines ;
    (v) les produits d'addition d'isocyanates et d'isothiocyanates sur les polyalkylènepolyamines ;
    (vi) les produits d'addition d'oxyde d'alkylène ou de polymères blocs polyoxyde d'alkylène sur les

polyalkylènepolyamines ;
(vii) les dérivés quaternisés de polyalkylène polyamines ;
(viii) les produits d'addition d'une silicone sur les polyalkylènepolyamines ;
(ix) un copolymère d'acide dicarboxylique et de polyalkylène polyamines ;

(B) les polyvinylimidazoles ;

(C) les polyvinylpyridines ;

(D) les produits d'addition de monomères 1-vinylimidazole de formule (I) :

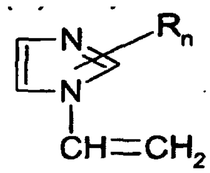
(I)

dans laquelle les radicaux R, identiques ou différents, représentent H, ou un radical alkyle en $C_1$-$C_6$, saturé ou insaturé, linéaire ou cyclique
n est un entier allant de 1 à 3,
avec les polyalkylènepolyamines (A)(i) à (A)(ix) ;

(E) les polymères à base d'acides aminés à chaîne latérale basique ;

(F) les dérivés réticulés des polymères (A)(i) à (A)(ix), (B) (C) (D) et (E).

2. Composition selon la revendication 1, **caractérisée par le fait que** le polymère polyaminé est choisi parmi ceux comprenant au moins 5% de fonctions amines tertiaires, avantageusement au moins 10% de fonctions amines tertiaires, et encore plus préférentiellement au moins 20%.

3. Composition selon l'une quelconque des revendications précédentes, **caractérisée par le fait que** le polymère polyaminé est choisi parmi :

(A)

(i) les polyéthylèneimines hyperbranchées comprenant au moins 5% de fonctions amines tertiaires;
(ii) les dérivés alkylés de polyéthylèneimine ;
(iii) les produits d'addition d'acides alkylcarboxyliques en C2-C30, saturés ou insaturés, linéaires ou ramifiés, sur la polyéthylèneimine ;
(iv) les produits d'addition de cétones et d'aldéhydes sur la polyéthylèneimine ;
(v) les produits d'addition d'isocyanates et de thioisocyanates sur la polyéthylèneimine ;
(vi) les produits d'addition d'oxyde d'alkylène ou de polymères blocs polyoxyde d'alkylène sur la polyéthyléneimine ;
(vii) les dérivés quatemisés de polyéthylèneimine ;
(viii) les produits d'addition d'une silicone sur la polyéthylèneimine ;
(ix) un copolymère d'acide dicarboxylique et de polyéthylèneimine ;

(B) les polyvinylimidazoles.

4. Composition selon l'une quelconque des revendications précédentes, **caractérisée par le fait que** le polymère polyaminé est choisi parmi :

(A) (i) les polyéthylèneimines hyperbranchées comprenant au moins 5% de fonctions amines tertiaires;
(A)(vi) les produits d'addition d'oxyde d'éthylène ou de polymères blocs polyoxyde d'éthylène sur la polyéthylèneimine.

5. Composition selon l'une quelconque des revendications précédentes, **caractérisée par le fait que** le polymère

polyaminé est choisi parmi les polyéthylèneimines hyperbranchées comprenant au moins 5% de fonctions amines tertiaires.

6. Composition selon l'une quelconque des revendications précédentes, **caractérisée par le fait que** le nanopigment est choisi parmi les nanopigments d'oxyde de titane.

7. Composition selon l'une quelconque des revendications précédentes, **caractérisée par le fait que** la concentration en nanopigments dans la composition est comprise entre 0,1 et 20 % en poids par rapport au poids total de la composition, et de préférence entre 0,25 et 15 %.

8. Composition selon l'une quelconque des revendications précédentes, **caractérisée par le fait que** la concentration en polymère polyaminé dans la composition est comprise entre 0,05 et 10 % en poids par rapport au poids total de la composition, préférentiellement entre 0,5 et 5%.

9. Composition selon l'une quelconque des revendications précédentes, **caractérisée par le fait qu'**elle comprend en outre au moins corps gras photo-oxydable.

10. Composition selon la revendication 9, **caractérisée par le fait qu'**elle comprend au moins un corps gras photo-oxydable choisi parmi ceux dont l'indice d'iode va de 5 à 200.

11. Composition selon l'une quelconque des revendications précédentes, **caractérisée par** le fait le taux d'insaturation C de la phase grasse de la composition, définie par la formule :

$$C = \sum_{i=1}^{n} [V.I.]_i \cdot [T.H]_i$$

dans laquelle [V.I.]i est l'indice d'iode de l'huile i et [T.H.]i est son pourcentage en poids par rapport au poids total de la phase grasse est compris entre 2,5 et 4000, et préférentiellement entre 50 et 4000.

12. Composition selon l'une quelconque des revendications précédentes, **caractérisée par le fait qu'**elle contient au moins une huile choisie parmi les huiles d'abricot, d'amande douce, d'arachide, d'avocat, de bancoulier, de bourrache, de camélia, de camélina, de carthame, de cassis, de céréales, de chouchou, de coco, de colza, de coriandre, de coton, de cumin, de cynara, d'onagre, de perilla, de foie de morue, de germe de mais, de jojoba, de kiwi, de lanoline, de lechti, de lin , de longan, de mangue, de noisette, d'olive, de palme, de passiflore, de pépins de raisin, de pin maritime, de pin parasol, de pistache, de rosier muscat, de sésame, de shoréa (graisse de sol), de soja, de son de riz, de tortue, de tournesol, de baleine, de thé, le beurre de karité les triglycérides de vitamine F.

13. Composition selon l'une quelconque des revendications précédentes, **caractérisée par le fait qu'**elle comprend de 0.5% à 60%, en poids par rapport au poids total de la composition, d'au moins un corps gras photo-oxydable.

14. Composition selon l'une quelconque des revendications précédentes, **caractérisée par le fait qu'**elle comprend en outre au moins une protéine ou un dérivé de protéine.

15. Composition selon l'une quelconque des revendications précédentes, **caractérisée par le fait qu'**elle comprend en outre au moins un agent complexant des métaux.

16. Composition selon la revendication précédente, **caractérisée par le fait que** l'agent complexant est choisi parmi l'acide 3-éthylènediamine tétra(méthylène phosphonique), l'acide diéthylènetriamine penta(méthylène phosphonique), l'acide diéthylènetriamine penta-acétique et leurs sels de sodium.

17. Composition selon l'une quelconque des revendications 15 et 16 précédentes, **caractérisée par le fait que** l'agent complexant des métaux est présent à une concentration allant de 0,005 % à 0,1 % en poids par rapport au poids total de la composition.

18. Composition dermatologique selon l'une quelconque des revendications précédentes destinée à traiter les signes du vieillissement cutané ou des cheveux, en particulier les signes du vieillissement induits par la photoperoxydation, et plus particulièrement les signes du vieillissement induits par la photoperoxydation du squalène et/ou du collagène.

**19.** Procédé de traitement cosmétique consistant à lutter contre ou à prévenir les signes du vieillissement induits par la photoperoxydation par application topique sur la peau et/ou le cuir chevelu et/ou les cheveux d'une composition selon l'une quelconque des revendications précédentes.

**20.** Procédé selon la revendication 19, consistant à lutter contre ou à prévenir les signes du vieillissement induits par la photoperoxydation du squalène et/ou du collagène.

**21.** Utilisation d'une composition selon l'une quelconque des revendications 1 à 18, comme composition cosmétique ou pour la fabrication d'une composition dermatologique protectrice de l'épiderme humain ou des cheveux contre les rayons ultraviolets, comme composition anti-solaire ou comme produit de maquillage.

**22.** Utilisation d'une composition selon l'une quelconque des revendications 1 à 18, comme composition cosmétique ou pour la fabrication d'une composition dermatologique destinée au traitement de lutte contre, et/ou de prévention de l'irritation, l'inflammation, l'immunosuppression et/ou l'acné photoinduits.

**23.** Procédé de conservation de compositions cosmétiques, pharmaceutiques, vétérinaires et/ou agroalimentaires renfermant au moins un lipide, une protéine ou un dérivé de protéine photo-oxydable, **caractérisé par le fait qu'**il consiste à incorporer au moins un polymère polyaminé tel que défini à la revendication 1 dans lesdites compositions.

**24.** Utilisation comme agent antioxydant d'un polymère polyaminé choisi parmi :

(A)

(i) les polyalkylène polyamines comprenant de 7 à 20000 motifs de répétition et comprenant au moins 5% d'amines tertiaires;
(ii) les produits d'addition d'acides alkylcarboxyliques sur les polyalkylènepolyamines ;
(iii) les produits d'addition de cétones et d'aldéhydes sur les polyalkylènepolyamines ;
(iv) les produits d'addition d'isocyanates et d'isothiocyanates sur les polyalkylènepolyamines ;
(v) les produits d'addition d'oxyde d'alkylène ou de polymères blocs polyoxyde d'alkylène sur les polyalkylènepolyamines ;
(vi) les dérivés quatemisés de polyalkylène polyamines ;
(vii) les produits d'addition d'une silicone sur les polyalkylènepolyamines ;
(viii) un copolymère d'acide dicarboxylique et de polyalkylène polyamines ;

(B) les polyvinylimidazoles ;
(C) les polyvinylpyridines ;
(D) les produits d'addition de monomères 1-vinylimidazole de formule (I) :

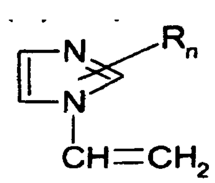

(I)

dans laquelle les radicaux R, identiques ou différents, représentent H, ou un radical alkyle en $C_1$-$C_6$, saturé ou insaturé, linéaire ou cyclique
n est un entier allant de 1 à 3,
avec les polyalkylènepolyamines (A)(i) à (A)(viii) ;
(E) les polymères à base d'acides aminés à chaîne latérale basique ;
(F) les dérivés réticulés des polymères (A)(i) à (A)(viii), (B) (C) (D) et (E).

**25.** Utilisation selon la revendication 24, dans une composition cosmétique ou pour la préparation d'une composition dermatologique destinée au traitement curatif ou préventif de l'irritation, l'inflammation, l'immunosuppression et/ou l'acné.

**26.** Utilisation selon l'une quelconque des revendications 24 ou 25, pour inhiber la peroxydation des lipides induite par les rayonnements UV.

**27.** Utilisation selon l'une quelconque des revendications 24 ou 25, pour inhiber la peroxydation des protéines et des dérivés de protéines induite par les rayonne**ments UV.**

**Patentansprüche**

**1.** Kosmetische und/oder dermatologische Zusammensetzung, die in einem kosmetisch oder dermatologisch akzeptablen Träger mindestens ein Nanopigment und mindestens ein Polyaminpolymer enthält, das ausgewählt ist unter:

(A)

(i) Polyalkylenpolyaminen, die 7 bis 20 000 wiederkehrende Einheiten und mindestens 5 % tertiäre Amine enthalten;
(ii) alkylierten Derivaten von Polyalkylenpolyaminen;
(iii) Additionsprodukten von Alkylcarbonsäuren und Polyalkylenpolyaminen;
(iv) Additionsprodukten von Ketonen und Aldehyden mit Polyalkylenpolyaminen;
(v) Additionsprodukten von Isocyanaten und Isothiocyanaten mit Polyalkylenpolyaminen;
(vi) Additionsprodukten von Alkylenoxid oder Polyalkylenoxid-Blockpolymeren mit Polyalkylenpolyaminen;
(vii) quaternisierten Derivaten von Polyalkylenpolyaminen;
(viii) Additionsprodukten von Siliconen und Polyalkylenpolyaminen;
(ix) Copolymeren von Dicarbonsäuren und Polyalkylenpolyaminen;

(B) Polyvinylimidazolen;
(C) Polyvinylpyridinen;
(D) Additionsprodukten von 1-Vinylimidazolmonomeren der Formel (I):

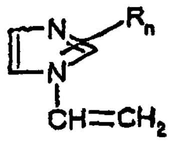

(I),

worin die Gruppen R, die gleich oder verschieden sind, H oder eine gesättigte oder ungesättigte, geradkettige oder cyclische $C_{1-6}$-Alkylgruppe bedeuten und n eine ganze Zahl von 1 bis 3 ist, mit Polyalkylenpolyaminen (A)(i) bis (A)(ix);
(E) Polymeren auf der Basis von Aminosäuren mit basischer Seitenkette; und
(F) vernetzten Derivaten der Polymere (A)(i) bis (A)(ix), (B), (C), (D) und (E).

**2.** Zusammensetzung nach Anspruch 1, **dadurch gekennzeichnet, dass** das Polyaminpolymer unter den Polymeren ausgewählt ist, die mindestens 5 % tertiäre Aminofunktionen, vorteilhaft mindestens 10 % tertiäre Aminofunktionen und noch bevorzugter mindestens 20 % tertiäre Aminofunktionen enthalten.

**3.** Zusammensetzung nach einem der vorhergehenden Ansprüche, **dadurch gekennzeichnet, dass** das Polyaminpolymer ausgewählt ist unter:

(A)

(i) hyperverzweigten Polyethyleniminen, die mindestens 5 % tertiäre Aminofunktionen enthalten,
(ii) alkylierten Derivaten von Polyethyleniminen,
(iii) Additionsprodukten von gesättigten oder ungesättigten, geradkettigen oder verzweigten Alkylcarbonsäuren mit 2 bis 30 Kohlenstoffatomen und Polyethylenimin;
(iv) Additionsprodukten von Ketonen und Aldehyden mit Polyethylenimin;
(v) Additionsprodukten von Isocyanaten und Isothiocyanaten und Polyethylenimin;
(vi) Additionsprodukten von Alkylenoxiden oder Polyalkylenoxid-Blockpolymere mit Polyethylenimin;
(vii) quaternisierten Derivaten von Polyethylenimin;
(viii) Additionsprodukten von Siliconen und Polyethylenimin;
(ix) Copolymeren von Dicarbonsäuren und Polyethylenimin;

(B) Polyvinylimidazolen.

**4.** Zusammensetzung nach einem der vorhergehenden Ansprüche, **dadurch gekennzeichnet, dass** das Polyaminpolymer ausgewählt ist unter:

(A)(i) hyperverzweigten Polyethyleniminen, die mindestens 5 % tertiäre Aminofunktionen enthalten;
A(vi) Additionsprodukten von Ethylenoxid oder Polyethylenoxid-Blockpolymeren mit Polyethylenimid.

**5.** Zusammensetzung nach einem der vorhergehenden Ansprüche, **dadurch gekennzeichnet, dass** das Polyaminpolymer unter den hyperverzweigten Polyethyleniminen ausgewählt ist, die mindestens 5 % tertiäre Aminofunktionen enthalten.

**6.** Zusammensetzung nach einem der vorhergehenden Ansprüche, **dadurch gekennzeichnet, dass** das Nanopigment unter den Nanopimenten von Titanoxid ausgewählt ist.

**7.** Zusammensetzung nach einem der vorhergehenden Ansprüche, **dadurch gekennzeichnet, dass** die Konzentration der Nanopigmente in der Zusammensetzung im Bereich von 0,1 1 bis 20 Gew.-%, bezogen auf das Gesamtgewicht der Zusammensetzung, und vorzugsweise 0,25 bis 15 % liegt,

**8.** Zusammensetzung nach einem der vorhergehenden Ansprüche, **dadurch gekennzeichnet, dass** die Konzentration des Polyaminpolymers in der Zusammensetzung im Bereich von 0,05 bis 10 Gew.-%, bezogen auf das Gesamtgewicht der Zusammensetzung, und vorzugsweise im Bereich von 0,5 bis 5 % liegt.

**9.** Zusammensetzung nach einem der vorhergehenden Ansprüche, **dadurch gekennzeichnet, dass** sie ferner mindestens eine durch Lichteinwirkung oxidierbare Fettsubstanz enthält.

**10.** Zusammensetzung nach Anspruch 9, **dadurch gekennzeichnet, dass** sie mindestens eine durch Lichteinwirkung oxidierbare Fettsubstanz enthält, die unter den Fettsubstanzen mit einem Iod-Index von 5 bis 200 ausgewählt ist.

**11.** Zusammensetzung nach einem der vorhergehenden Ansprüche, **dadurch gekennzeichnet, dass** der Ungesättigtheitsgrad C der Fettphase der Zusammensetzung, der durch die folgende Formel definiert ist:

$$C = \sum_{i=1}^{n} [V.I.]_i \cdot [T.H]_i$$

worin [V.I.]i der Iod-Index des Öls i ist und [T.H.]i seinen prozentualen Gewichtsanteil, bezogen auf das Gesamtgewicht der Fettphase, bedeutet, im Bereich von 2,5 bis 4 000 und vorzugsweise 50 bis 4 000 liegt.

**12.** Zusammensetzung nach einem der vorhergehenden Ansprüche, **dadurch gekennzeichnet, dass** sie ferner mindestens ein Öl enthält, das unter Aprikosenkernöl, Süßmandelöl, Erdnussöl, Avocadoöl, Kukuinussöl, Borretschöl, Kamelienöl, Leindotteröl, Distelöl, Johanniskernöl, Getreidekeimöl, Stachelgurkenöl, Kokosöl, Rapsöl, Korianderöl, Baumwollsamenöl, Kümmelöl, Artischockenöl, Nachtkerzenöl, Perillaöl, Dorschleberöl, Maiskeimöl, Jojobaöl, Kiwiöl, Lanolinöl, Litchiöl, Leinöl, Longanöl, Mangoöl, Haselnussöl, Olivenöl, Palmöl, Passionsblumenöl, Traubenkernöl, Strandkieferöl, Steinkieferöl, Pistazienkernöl, Wildrosenöl, Sesamöl, Shoreaöl, Illipe-Butter, Sojaöl, Reiskeimöl, Schildkrötenöl, Sonnenblumenöl, Walrat, Teeöl, Sheabutter und den Triglyceriden von Vitamin F ausgewählt ist.

**13.** Zusammensetzung nach einem der vorhergehenden Ansprüche, **dadurch gekennzeichnet, dass** sie 0,5 bis 60

Gew.-% mindestens einer durch Lichteinwirkung oxidierbaren Fettsubstanz, bezogen auf das Gesamtgewicht der Zusammensetzung, enthält.

**14.** Zusammensetzung nach einem der vorhergehenden Ansprüche, **dadurch gekennzeichnet, dass** sie ferner mindestens ein Protein oder Proteinderivat enthält.

**15.** Zusammensetzung nach einem der vorhergehenden Ansprüche, **dadurch gekennzeichnet, dass** sie außerdem mindestens einen Metallkomplexbildner enthält.

**16.** Zusammensetzung nach dem vorhergehenden Anspruch, **dadurch gekennzeichnet, dass** der Komplexbildner unter 3-Ethylendiamintetra(methylen-phosphonsäure), Diethylentriaminpenta(methylen-phosphonsäure), Diethylentriaminpentaessigsäure und deren Natriumsalzen ausgewählt ist.

**17.** Zusammensetzung nach einem der vorhergehenden Ansprüche 15 und 16, **dadurch gekennzeichnet, dass** der Metallkomplexbildner in einer Konzentration von 0,005 bis 1 Gew.-%, bezogen auf das Gesamtgewicht der Zusammensetzung, enthalten ist.

**18.** Dermatologische Zusammensetzung nach einem der vorhergehenden Ansprüche, die dazu vorgesehen ist, die Anzeichen der Hautalterung oder der Alterung der Haare zu behandeln, insbesondere die Anzeichen der Alterung, die durch Photoperoxidation ausgelöst werden und besonders die durch Photoperoxidation von Squalen und/oder Collagen verursachten Anzeichen der Hautalterung.

**19.** Verfahren zur kosmetischen Behandlung, das darin besteht, die durch Photoperoxidation induzierten Anzeichen der Hautalterung zu bekämpfen oder ihnen vorzubeugen, indem eine Zusammensetzung nach einem der vorhergehenden Ansprüche auf die Haut und/oder die Kopfhaut und/oder die Haare topisch angewandet wird.

**20.** Verfahren nach Anspruch 19, das darin besteht, die durch Photoperoxidation von Squalen und/oder Collagen induzierten Anzeichen der Hautalterung zu bekämpfen oder ihnen vorzubeugen.

**21.** Verwendung einer Zusammensetzung nach einem der Ansprüche 1 bis 18 als kosmetische Zusammensetzung oder zur Herstellung einer dermatologischen Zusammensetzung zum Schutz der menschlichen Epidermis oder der Haare vor UV-Strahlung, als Sonnenschutzmittel oder als Produkt zum Schminken.

**22.** Verwendung einer Zusammensetzung nach einem der Ansprüche 1 bis 18 als kosmetische Zusammensetzung oder zur Herstellung einer dermatologischen Zusammensetzung, die dazu vorgesehen ist, Irritationen, Entzündungen, Immunsuppression und/oder Akne, welche photoinduziert sind, zu bekämpfen und/ oder ihnen vorzubeugen.

**23.** Verfahren zur Konservierung von kosmetischen Zusammensetzungen, pharmazeutischen Zusammensetzungen, veterinärmedizinischen Zusammensetzungen und/oder in der Lebensmittelindustrie verwendeten Zusammensetzungen, die mindestens ein Lipid, ein Protein oder ein Proteinderivat enthalten, die durch Lichteinwirkung oxidierbar sind, **dadurch gekennzeichnet, dass** es darin besteht, mindestens ein Polyaminpolymer nach Anspruch 1 in diese Zusammensetzungen einzuarbeiten.

**24.** Verwendung eines Polyaminpolymers als Antioxidationsmittel, das unter den folgenden Verbindungen ausgewählt ist:

(A)

(i) Polyalkylenpolyaminen die 7 bis 20 000 wiederkehrende Einheiten enthalten und mindestens 5 % tertiäre Amine aufweisen;
(ii) Additionsprodukten von Alkylcarbonsäuren und Polyalkylenpolyaminen;
(iii) Additionsprodukten von Ketonen und Aldehyden mit Polyalkylenpolyaminen;
(iv) Additionsprodukten von Isocyanaten und Isothiocyanaten mit Polyalkylenpolyaminen;
(v) Additionsprodukten von Alkylenoxid oder Polyalkylenoxid-Blockpolymeren mit Polyalkylenpolyaminen;
(vi) quaternisierten Derivaten von Polyalkylenpolyaminen;
(vii) Additionsprodukten von Siliconen mit Polyalkylenpolyaminen;
(viii) Copolymeren von Dicarbonsäuren und Polyalkylenpolyaminen;

(B) Polyvinylimidazolen;
(C) Polyvinylpyridinen;
(D) Additionsprodukten von 1-Vinylimidazolmonomeren der Formel (I):

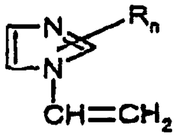

(I),

worin die Gruppen R, die gleich oder verschieden sind, H oder eine gesättigte oder ungesättigte, geradkettige oder cyclische $C_{1-6}$-Alkylgruppe bedeuten und n eine ganze Zahl von 1 bis 3 ist, mit Polyalkylenpolyaminen (A)(i) bis (A)(viii);
(E) Polymeren auf der Basis von Aminosäuren mit basischer Seitenkette; und
(F) vernetzten Derivaten von Polymeren (A)(i) bis (A)(viii), (B), (C), (D) und (E).

**25.** Verwendung nach Anspruch 24 in einer kosmetischen Zusammensetzung oder zur Herstellung einer dermatologischen Zusammensetzung, die für die kurative oder präventive Behandlung von Irritationen, Entzündungen, Immunsuppression und/oder Akne vorgesehen ist.

**26.** Verwendung nach einem der Ansprüche 24 oder 25 zur Inhibierung der Peroxidation von Lipiden, die durch UV-Strahlung ausgelöst wird.

**27.** Verwendung nach einem der Ansprüche 24 oder 25 zur Inhibierung der Peroxidation von Proteinen und Proteinderivaten, die durch UV-Strahlung ausgelöst wird.

## Claims

**1.** Cosmetic and/or dermatological composition comprising, in a cosmetically or dermatologically acceptable support, at least one nanopigment, and at least one polyamino polymer chosen from:

(A)

(i) polyalkylenepolyamines comprising from 7 to 20,000 repeating units and comprising at least 5% of tertiary amines;
(ii) alkyl derivatives of polyalkylenepolyamines;
(iii) the addition products of alkylcarboxylic acids with polyalkylenepolyamines;
(iv) the addition products of ketones and aldehydes with polyalkylenepolyamines;
(v) the addition products of isocyanates and isothiocyanates with polyalkylenepolyamines;
(vi) the addition products of alkylene oxide or of polyalkylene oxide block polymers with polyalkylenepolyamines;
(vii) quaternized derivatives of polyalkylenepolyamines;
(viii) the addition products of a silicone with polyalkylenepolyamines;
(ix) a copolymer of dicarboxylic acid and of polyalkylenepolyamines;

(B) polyvinylimidazoles;
(C) polyvinylpyridines;
(D) the addition products of 1-vinylimidazole monomers of formula (I):

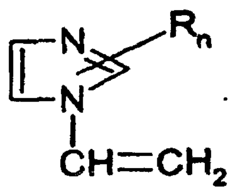

(I)

in which the radicals R, which may be identical or different, represent H or a linear or cyclic, saturated or unsaturated $C_1$-$C_6$ alkyl radical,
n is an integer ranging from 1 to 3,
with polyalkylenepolyamines (A)(i) to (A)(ix);
(E) polymers based on amino acids with a basic side chain;
(F) crosslinked derivatives of the polymers (A)(i) to (A) (ix) , (B), (C), (D) and (E).

2. Composition according to Claim 1, **characterized in that** the polyamino polymer is chosen from those comprising at least 5% of tertiary amine functions, advantageously at least 10% of tertiary amine functions and even more preferably at least 20%.

3. Composition according to either of the preceding claims, **characterized in that** the polyamino polymer is chosen from:

(A)

(i) hyperbranched polyethyleneimines comprising at least 5% of tertiary amine functions;
(ii) alkyl derivatives of polyethyleneimine;
(iii) the addition products of linear or branched, saturated or unsaturated C2-C30 alkylcarboxylic acids with polyethyleneimine;
(iv) the addition products of ketones and aldehydes with polyethyleneimine;
(v) the addition products of isocyanates and isothiocyanates with polyethyleneimine;
(vi) the addition products of alkylene oxide or of polyalkylene oxide block polymers with polyethyleneimine;
(vii) quaternized derivatives of polyethyleneimine;
(viii) the addition products of a silicone with polyethyleneimine;
(ix) a copolymer of dicarboxylic acid and of polyethyleneimine;

(B) polyvinylimidazoles.

4. Composition according to any one of the preceding claims, **characterized in that** the polyamino polymer is chosen from:

(A) (i) hyperbranched polyethyleneimines comprising at least 5% of tertiary amine functions;
(A) (vi) the addition products of ethylene oxide or of polyethylene oxide block polymers with polyethyleneimine.

5. Composition according to any one of the preceding claims, **characterized in that** the polyamino polymer is chosen from hyperbranched polyethyleneimines comprising at least 5% of tertiary amine functions.

6. Composition according to any one of the preceding claims, **characterized in that** the nanopigment is chosen from titanium oxide nanopigments.

7. Composition according to any one of the preceding claims, **characterized in that** the concentration of nanopigments in the composition is between 0.1 and 20% by weight relative to the total weight of the composition, and preferably between 0.25 and 15%.

8. Composition according to any one of the preceding claims, **characterized in that** the concentration of polyamino polymer in the composition is between 0.05 and 10% by weight relative to the total weight of the composition, preferably between 0.5 and 5%.

**9.** Composition according to any one of the preceding claims, **characterized in that** it also comprises at least one photo-oxidizable fatty substance.

**10.** Composition according to Claim 9, **characterized in that** it comprises at least one photo-oxidizable fatty substance chosen from those whose iodine value ranges from 5 to 200.

**11.** Composition according to any one of the preceding claims, **characterized in that** the degree of unsaturation C of the fatty phase of the composition, defined by the formula:

$$C = \sum_{i=1}^{n} [V.I.]i*[T.H]i$$

in which [V.I.]i is the iodine value of the oil i and [T.H.]i is its weight percentage relative to the total weight of the fatty phase, is between 2.5 and 4000 and preferably between 50 and 4000.

**12.** Composition according to any one of the preceding claims, **characterized in that** it contains at least one oil chosen from apricot oil, sweet almond oil, groundnut oil, avocado oil, candlenut oil, borage oil, camellia oil, false flax oil, safflower oil, blackcurrant oil, cereal oil, chayote oil, coconut oil, rapeseed oil, coriander oil, cotton oil, cumin oil, cynara oil, evening primrose oil, perilla oil, cod liver oil, corn germ oil, jojoba oil, kiwi oil, lanolin oil, lychee oil, linseed oil, longan oil, mango oil, hazelnut oil, olive oil, palm oil, passionflower oil, grapeseed oil, cluster pine oil, Italian stone pine oil, pistachio oil, musk rose oil, sesame oil, shorea oil (floor grease), soybean oil, rice bran oil, turtle oil, sunflower oil, whale oil, tea oil, karite butter and vitamin F triglycerides.

**13.** Composition according to any one of the preceding claims, **characterized in that** it comprises from 0.5% to 60% by weight, relative to the total weight of the composition, of at least one photo-oxidizable fatty substance.

**14.** Composition according to any one of the preceding claims, **characterized in that** it also comprises at least one protein or a protein derivative.

**15.** Composition according to any one of the preceding claims, **characterized in that** it also comprises at least one metal-complexing agent.

**16.** Composition according to the preceding claim, **characterized in that** the complexing agent is chosen from 3-ethylenediaminetetra(methylenephosphonic acid), diethylenetriaminepenta(methylenephosphonic acid) and diethylenetriaminepentaacetic acid, and the sodium salts thereof.

**17.** Composition according to either of the preceding Claims 15 and 16, **characterized in that** the metal-complexing agent is present at a concentration ranging from 0.005% to 0.1% by weight relative to the total weight of the composition.

**18.** Dermatological composition according to any one of the preceding claims, which is intended to treat the signs of ageing of the skin or the hair, in particular the signs of ageing induced by photo-peroxidation, and more particularly the signs of ageing induced by the photo-peroxidation of squalene and/or collagen.

**19.** Cosmetic treatment process which consists in combating or preventing the signs of ageing induced by photo-peroxidation by topical application, to the skin and/or the scalp and/or the hair, of a composition according to any one of the preceding claims.

**20.** Process according to Claim 19, which consists in combating or preventing the signs of ageing induced by the photo-peroxidation of squalene and/or collagen.

**21.** Use of a composition according to any one of Claims 1 to 18, as a cosmetic composition or for the manufacture of a dermatological composition for protecting the human epidermis or the hair against ultraviolet rays, as an antisun composition or as a make-up product.

**22.** Use of a composition according to any one of Claims 1 to 18, as a cosmetic composition or for the manufacture of a dermatological composition intended for treatment to combat and/or prevent light-induced irritation, inflammation, immunosuppression and/or acne.

**23.** Process for conserving cosmetic, pharmaceutical, veterinary and/or agrifood compositions containing at least one photo-oxidizable lipid, protein or protein derivative, **characterized in that** it consists in incorporating at least one polyamino polymer as defined in Claim 1 into the said compositions.

**24.** Use, as an antioxidant, of a polyamino polymer chosen from:

(A)

(i) polyalkylenepolyamines comprising from 7 to 20,000 repeating units and comprising at least 5% of tertiary amines;
(ii) the addition products of alkylcarboxylic acids with polyalkylenepolyamines;
(iii) the addition products of ketones and aldehydes with polyalkylenepolyamines;
(iv) the addition products of isocyanates and isothiocyanates with polyalkylenepolyamines;
(v) the addition products of alkylene oxide or of polyalkylene oxide block polymers with polyalkylenepolyamines;
(vi) quaternized derivatives of polyalkylenepolyamines;
(vii) the addition products of a silicone with polyalkylenepolyamines;
(viii) a copolymer of dicarboxylic acid and of polyalkylenepolyamines;

(B) polyvinylimidazoles;
(C) polyvinylpyridines;
(D) the addition products of 1-vinylimidazole monomers of formula (I):

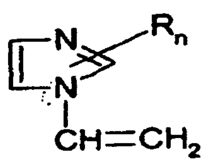

(I)

in which the radicals R, which may be identical or different, represent H or a linear or cyclic, saturated or unsaturated $C_1$-$C_6$ alkyl radical,
n is an integer ranging from 1 to 3,
with polyalkylenepolyamines (A)(i) to (A)(viii);
(E) polymers based on amino acids with a basic side chain;
(F) crosslinked derivatives of the polymers (A)(i) to

(A) (viii), (B), (C), (D) and (E).

**25.** Use according to Claim 24, in a cosmetic composition or for the preparation of a dermatological composition intended for the curative or preventive treatment of irritation, inflammation, immunosuppression and/or acne.

**26.** Use according to either of Claims 24 and 25, for inhibiting the peroxidation of lipids which is induced by UV radiation.

**27.** Use according to either of Claims 24 and 25, for inhibiting the peroxidation of proteins and protein derivatives which is induced by UV radiation.

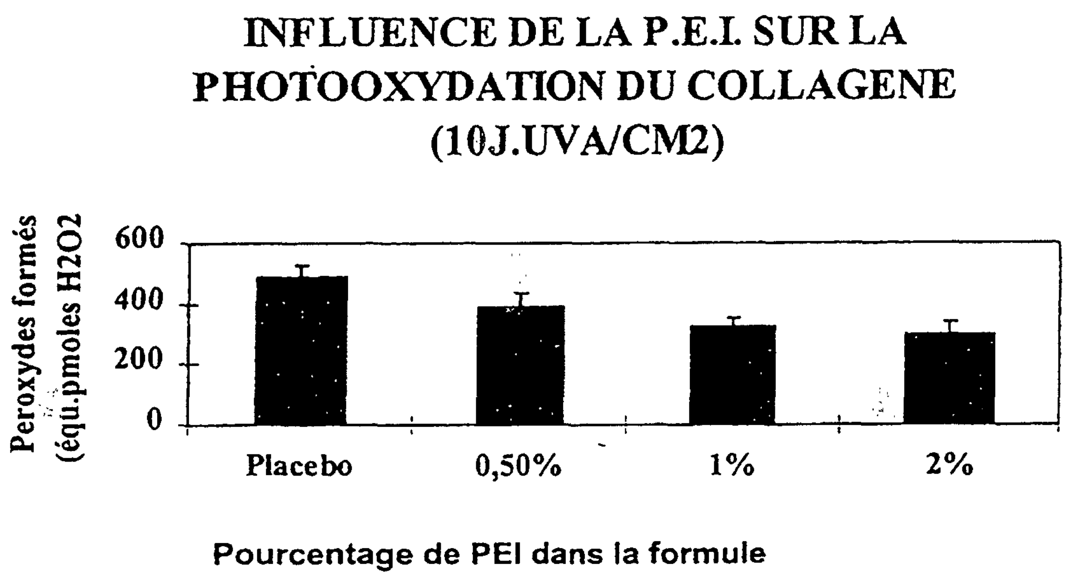
INFLUENCE DE LA P.E.I. SUR LA
PHOTOOXYDATION DU COLLAGENE
(10J.UVA/CM2)
Peroxydes formés
(équ.pmoles H2O2
600
400
200
0
Placebo
0,50%
1%
2%
Pourcentage de PEI dans la formule

FIGURE 1